# EUROPÄISCHE PATENTSCHRIFT

(11) **EP 0 988 833 B1**
(45) Veröffentlichungstag und Bekanntmachung des Hinweises auf die Patenterteilung: **01.10.2003**
(21) Anmeldenummer: 99810742.9
(22) Anmeldetag: 18.08.1999
(51) Int. Cl.: A61B 17/80

(54) **Osteosyntheseplatte mit mehreren Knochenschrauben**
Osteosynthesis plate with multiple bone screws
Plaque d'ostéosynthèse avec plusieurs vis à os

(30) Priorität: 24.09.1998 EP 98810957
(43) Veröffentlichungstag der Anmeldung: 29.03.2000
(73) Patentinhaber: Centerpulse Orthopedics Ltd., 6340 Baar (CH)
(72) Erfinder: Studer, Armin, 6312 Steinhausen (CH); Donno, Cosimo, 8400 Winterthur (CH); Fröhlich, Markus, 8362 Balterswil (CH); Gnos, Robert, 3177 Laupen (CH)
(74) Vertreter: Manitz, Finsterwald & Partner Gbr

(56) Entgegenhaltungen:
- EP-A- 0 807 420
- WO-A-98/34554

## Beschreibung

Die Erfindung handelt von einer Osteosyntheseplatte mit mehreren Knochenschrauben, welche einen Kopf mit kugelförmiger Anlagefläche aufweisen, um beim Eindrehen in einem vorgegebenen Winkelbereich α unter einem Schrägwinkel auf einer ringförmigen, annähernd sphärischen Fläche der Osteosyntheseplatte anzuliegen, und mit einer Sicherungsschraube, welche in der Richtung auf die ringförmige sphärische Fläche mit einem Aussengewinde in der Osteosyntheseplatte einschraubbar ist, um den Kopf der Knochenschraube dort anzupressen.

Osteosyntheseplatten werden als Implantate an Knochenteilen mit mehreren Knochenschrauben befestigt, wobei die Dauer für den Verbleib der Platte in der Grössenordnung von Wochen oder Jahren liegen kann. Da sich an Knochenschrauben wie an anderen Knochenimplantaten die Vorspannung, welche mit einer Primärverankerung im Knochen erreicht wird, über Jahre hinweg abbauen kann, ist eine nur als Zuganker ausgeführte Knochenschraube zur Befestigung einer versteifenden Osteosyntheseplatte mit dem Nachteil verbunden, dass vom Schraubenkopf kaum Biegemomente auf die Platte übertragbar sind.

In der DE-A-195 42 116 ist eine platzsparende Befestigung zwischen einer Knochenschraube und einem flachen Implantatkörper gezeigt. Eine Sicherungsschraube mit einer kalottenförmigen Lagerfläche presst gegen einen kugelförmigen Abschnitt des Kopfes der Knochenschraube und bewirkt ein Klemmen eines weiteren kugelförmigen Abschnitts mit gleichem Kugelmittelpunkt auf einer sphärischen Schulter des Implantatkörpers. Ein Nachteil dieser Anordnung besteht darin, dass extrem hohe Anpresskräfte in der Richtung der Achse der Befestigungsschraube aufgebracht werden müssen, um vernüftige Biegemomente von der Knochenschraube auf die Platte übertragen zu können. Da die Uebertragung der Kräfte im wesentlichen auf Reibschluss mittels axialer Zustellung der Sicherungsschraube beruht, bestehen die auf Reibung beruhenden Unsicherheiten und Streuungen bei der Uebertragung von Biegemomenten.

Dokument EP-A-0807 420 offenbart eine Vorrichtung gemäß dem Oberbegriff des Anspruchs 1.

Aufgabe der Erfindung ist es, die Befestigung zwischen knochenschrauben und Osteosyntheseplatte, insbesondere die Biegesteifigkeit, zu verbessern. Dies wird gemäss Anspruch 1 dadurch erreicht, dass die Sicherungsschraube eine konische Vertiefung mit einem Innengewinde aufweist, welches in der gleichen Richtung wie das Aussengewinde verläuft und die gleiche Steigung besitzt, um mit dem Innengewinde in seiner eigenen Spur eine Gewinderille am Kopf der Knochenschraube zu erzeugen, was wesentlich zur Erhöhung der Biegesteifigkeit zwischen Knochenschraube und Osteosyntheseplatte beiträgt.

Die Erfindung hat den Vorteil, dass mit der Zustellung der Sicherungsschraube die Gewindespitzen des Innengewindes relativ zum Kopf der Knochenschraube ihre Höhe nicht verändern, weil Aussen- und Innengewinde der Sicherungsschraube gleiche Drehrichtung und gleiche Steigung s besitzen, dass hingegen die Gewindespitzen radial und schleifend in die Kugelfläche eindringen und eine Gewinderille erzeugen, die formschlüssig bezüglich Biegebelastungen mit der Sicherungsschraube verbunden ist. Die Sicherungsschraube ihrerseits ist bezüglich dieser Biegebelastung ebenfalls formschlüssig über ihr Aussengewinde mit der Platte verbunden. Auf diese Weise wird ein Formschluss bezüglich Biegebelastungen zwischen Knochenschraube und Platte erreicht, ohne darauf verzichten zu müssen, dass die Einschraubrichtung der Knochenschraube in einem Winkelbereich α von 90° abweichend gewählt werden kann. In den abhängigen Ansprüchen 2 bis 12 sind vorteilhafte Weiterbildungen der Erfindung aufgeführt. Mit einem mehrgängigen Innengewinde wird die Anzahl der am Formschluss beteiligten Gewindespitzen erhöht. Wenn der Werkstoff der Sicherungsschraube im Bereich des Innengewindes härter als der Kopf der Knochenschraube ist, können die Gewinderillen leichter eingeformt werden. Als eine weitere Massnahme können quer zu den Gewindespitzen am Innengewinde Nuten angebracht sein, die es selbstschneidend machen. Dadurch dass der Kopf der Knochenschraube nur mit einem Kugeldurchmesser ausgeführt ist, kann die gleiche Kugeloberfläche - je nach Schrägwinkel der Schraubenachse - mit der Sicherungsschraube oder mit der ringförmigen Abstützfläche an der Platte im Eingriff sein. Der Eingriffsdurchmesser zur Sicherungsschraube ist somit auf einem Maximum und es entsteht ein grosser Hebelarm zur Uebertragung des Biegemomentes in den formschlüssigen Flächen. Dies gestattet es, die Verbindung weiter bezüglich Baugrösse zu optimieren, d.h. zu verkleinern. Der Kugelkopf der Knochenschraube kann einen Durchmesser aufweisen, der weniger als 40 % grösser als der Gewindedurchmesser der Knochenschraube ist. Der halbe Konuswinkel β der Vertiefung an der Sicherungsschraube kann kleiner 20° z.B. auf 15° gehalten werden. Für den Selbsthalt der Gewinde an der Sicherungsschraube wird eine Steigung s ≤ 1 mm z.B. S = 0,75 mm gewählt.

Da die Knochenschraube Biegekräfte, sowie Zug- und Druckkräfte in der Plattenebene übertragen kann, besteht die Möglichkeit, die Auflagefläche von der Platte zum Knochen, an dem sie anliegen soll, zu reduzieren, beispielsweise auf einen leicht vorstehenden Kragen an der Unterseite der Platte, oder die Platte überhaupt nicht aufliegen zu lassen. Dies hat den Vorteil, dass die Durchblutung und somit die langfristige Heilung am Knochen verbessert wird. Eine weitere Massnahme besteht darin, die Zwischenstücke der Platte zwischen den einzelnen Knochenschrauben so zu schwächen, dass sie plastisch im Operationssaal verbogen werden können, um sie an die Knochenform anzupassen. Dazu können zwei Einschraubwerkzeuge, welche das gleiche Aussengewinde wie die Sicherungsschraube aufweisen, in zwei benachbarten Schraubenaufnahmen der Platte eingeschraubt werden und die Platte im geschwächten Zwischenstück in eine gewünschte Richtung abgebogen oder verdreht werden.

Eine besondere Operationstechnik ergibt sich, wenn die Distanz der Platte mit einschraubbaren Distanzstücken zum Knochen bestimmt wird, die Knochenschrauben eingeschraubt und mit Sicherungsschrauben in der Platte verankert werden, und anschliessend die Distanzstücke entfernt werden. Als Distanzstücke eignen sich Schrauben mit einem Gewinde wie die Sicherungsschrauben, welche in nicht besetzten Schraubenaufnahmen der Platte eingeschraubt werden und mit einem Stempel über die Unterseite der Platte vorstehen.

Im weiteren ist die Erfindung anhand von Ausführungsbeispielen beschrieben. Es zeigen:
- Fig. 1: schematisch einen Teilschnitt durch eine Osteosyntheseplatte mit einer erfindungsgemässen Abstützung einer Knochenschraube;
- Fig.2: schematisch eine vergrösserte Draufsicht auf die Sicherungsschraube aus Figur 1;
- Fig.3: schematisch einen vergrösserten Längsschnitt durch die Sicherungsschraube von Figur 1;
- Fig.4: schematisch einen stark vergrösserten Ausschnitt von Figur 3,
- Fig.5: schematisch einen vergrösserten Ausschnitt einer Osteosyntheseplatte mit zwei Knochenschrauben;
- Fig.6: schematisch im vergrösserten Schnitt als Explosionszeichnung ein weiteres Ausführungsbeispiel einer erfindungsgemässen Verbindung von Knochenschraube und Osteosyntheseplatte;
- Fig.7 u. 8: schematisch und vergrössert ein einschraubbares Biegewerkzeug mit den Gewindeabmessungen des Aussengewindes der Sicherungsschraube, um ein schwächeres Zwischenstück zwischen zwei Schraubenbefestigungen zu verbiegen, und
- Fig. 9: schematisch und vergrössert ein einschraubbares Distanzstück, welches nach dem Eindrehen von benachbarten Knochenschrauben entfernbar ist.

Die Figuren zeigen eine Osteosyntheseplatte mit mehreren Knochenschrauben, deren kugelförmiger Kopf auf einer ringförmigen, annähernd sphärischen Fläche der Platte in unterschiedlichen Winkelstellungen anliegen kann, und zugehörige Sicherungsschrauben, die in ihrer axialen Richtung auf den Kugelkopf pressen. Die Sicherungsschrauben sind mit einer konischen Vertiefung versehen, die ein Innengewinde aufweist, welches in der gleichen Richtung wie ihr Aussengewinde verläuft und die gleiche Steigung s wie das Aussengewinde besitzt, um radial eine Gewinderille am Kugelkopf einzuschneiden, welche einen Formschluss zur Uebertragung grosser Biegekäfte von Knochenschraube auf Sicherungsschraube und Platte erzeugt.

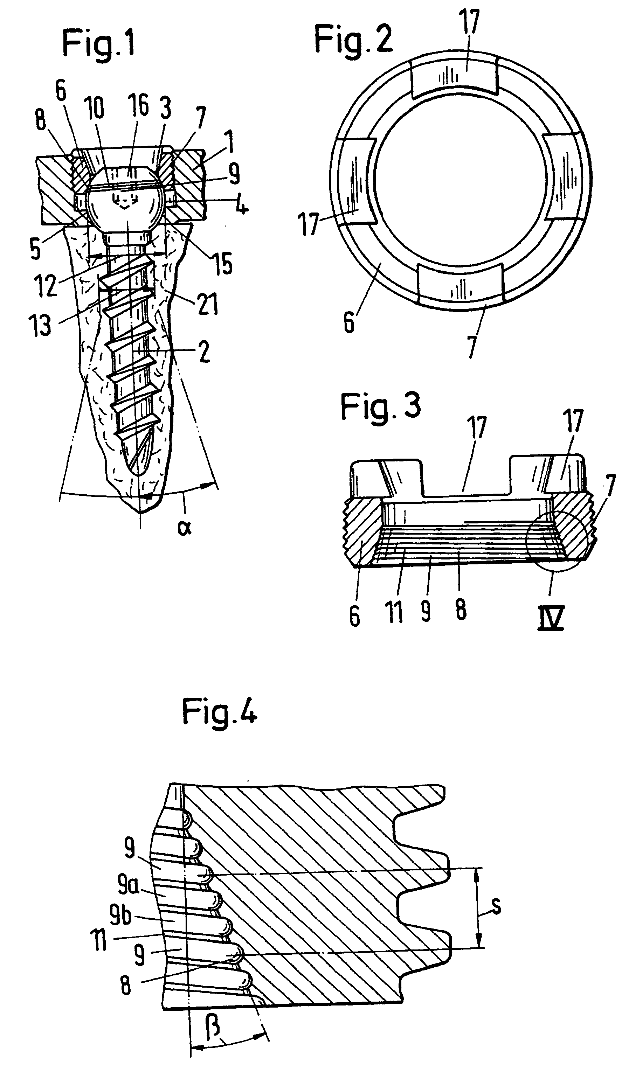

Im Ausführungsbeispiel der Figuren 1 bis 5 wid eine Osteosyntheseplatte 1 auf einem Knochen 21 aufgelegt und eine Knochenschraube 2 innerhalb eines Winkelbereichs α in einem passenden Winkel mit einem Schraubendreher (hier nicht gezeigt) an ihrem Innensechskant 16 eingedreht, bis der kugelförmige Schraubenkopf 3 an einer ringförmigen, annähernd sphärischen Fläche 5 anliegt und über einen vorstehenden Kragen 15 eine vorgesehene Vorspannung zum Knochen 21 erzeugt. Anschliessend wird die Sicherungsschraube 6, die Ausnehmungen 17 zur Drehmomentübertragung aufweist, mit einem Werkzeug eingedreht, bis sie abgestützt an ihrem Aussengewinde auf dem kugelförmigen Schraubenkopf 3 aufsitzt. Als Kontakfläche ist an der Sicherungsschraube 2 eine konische Vertiefung 8 vorgesehen, die ein Innengewinde 9 aufweist, welches die gleiche Richtung und die gleiche Steigung s wie das Aussengewinde 7 aufweist. Beim Weiterdrehen der Sicherungsschraube 6 dringen die Gewindespitzen 11 radial in den Kugelkopf 3 ein und schneiden Gewinderillen 10, welche mit den Gewindespitzen 11 einen hervorragenden Formschluss zur Uebertragung von Biegemomenten an einem Kugelkopf bilden.

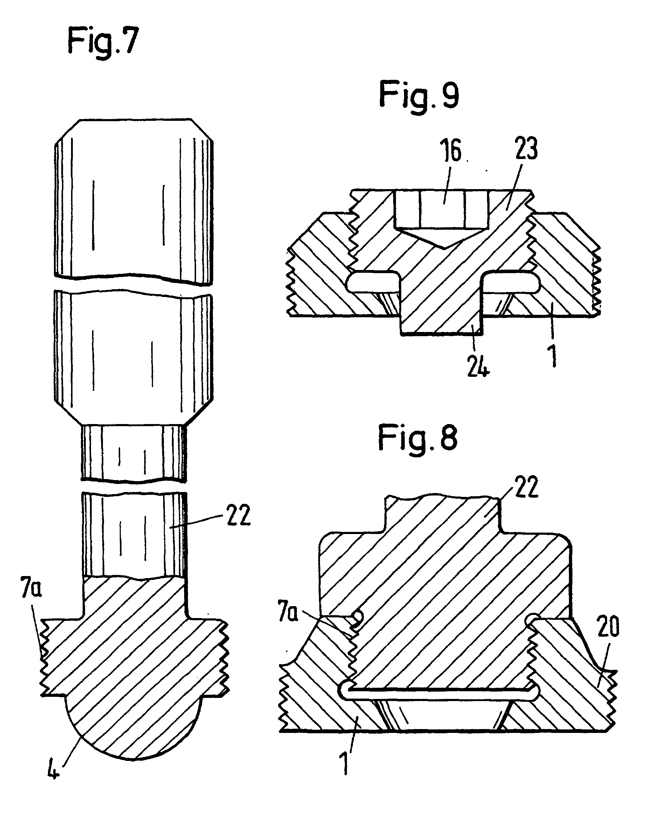

Bei einer weiteren Anwendung der Ausführung nach den Figuren 1 bis 5 werden die Knochenschrauben nur so weit eingedreht, dass zwischen Platte und Knochen ein Abstand erhalten bleibt und anschliessend die Knochenschrauben 2 in der Platte 1 mit Sicherungsschrauben gesichert. Als kurzzeitige Distanzhalter sind dabei Distanzschrauben 23 mit einem über die Plattenunterseite vorstehenden Stempel 24 (siehe Figur 9) vorgesehen. Diese werden an den unbesetzten Schraubenaufnahmen der Platte 1 eingeschraubt, um die Distanz zum Knochen zu halten, bis die Knochenschrauben 2 eingeschraubt und teilweise fixiert sind. In diesem Zustand ist noch eine Justierung an den Knochenschrauben für das endgültige Reponieren von Knochenteilen möglich. Anschliessend werden die Sicherungsschrauben 2 angezogen und die Distanzschrauben 23 entfernt.

In Figur 4 erkennt man, dass das Innengewinde dreigängig mit den Gewindegängen 9, 9a, 9b ausgeführt ist. Der halbe Konuswinkel β der konischen Vertiefung 8 beträgt 15°. Auf diese Weise sind auch bei einem kleinen Kugelkopf 3 von 6 mm Durchmesser mit beispielsweise 2- oder 3-gängigen Innengewinden 9 genügend Gewindespitzen im Eingriff, um einen Formschluss zu bilden. Dadurch dass der halbe Konuswinkel β klein gewählt ist, heben sich die radialen Kräfte beim Erzeugen der Gewinderillen 11 auf. Es entsteht lediglich eine grosse Ringspannung in der Sicherungsschraube 3. Die Sicherungsschraube 3 besitzt nach oben einen konischen und relativ grossen Durchbruch und an ihrer Peripherie Aussparungen 17. Dies gestattet es, mit einem ringförmigen Werkzeug in die Aussparungen 17 einzugreifen, um die Sicherungsschraube anzuziehen, und gleichzeitig mit einem zweiten Werkzeug durch den Ring hindurch am Innensechskant 16 einzugreifen, um das am Schraubenkopf 3 erzeugte Drehmoment zu kompensieren. Die Kanten, welche die Aussparungen 17 begrenzen, und die Einläufe der Gewinde 7, 9 sind verrundet, damit der Operateur keine Verletzungen an seinen Gummihandschuhen erzeugt.

Der Kugelkopf 3 der Sicherungsschraube hat einen Durchmesser 12, der um weniger als 40 % den Aussendurchmesser 13 des Schraubengewindes 14 überragt.

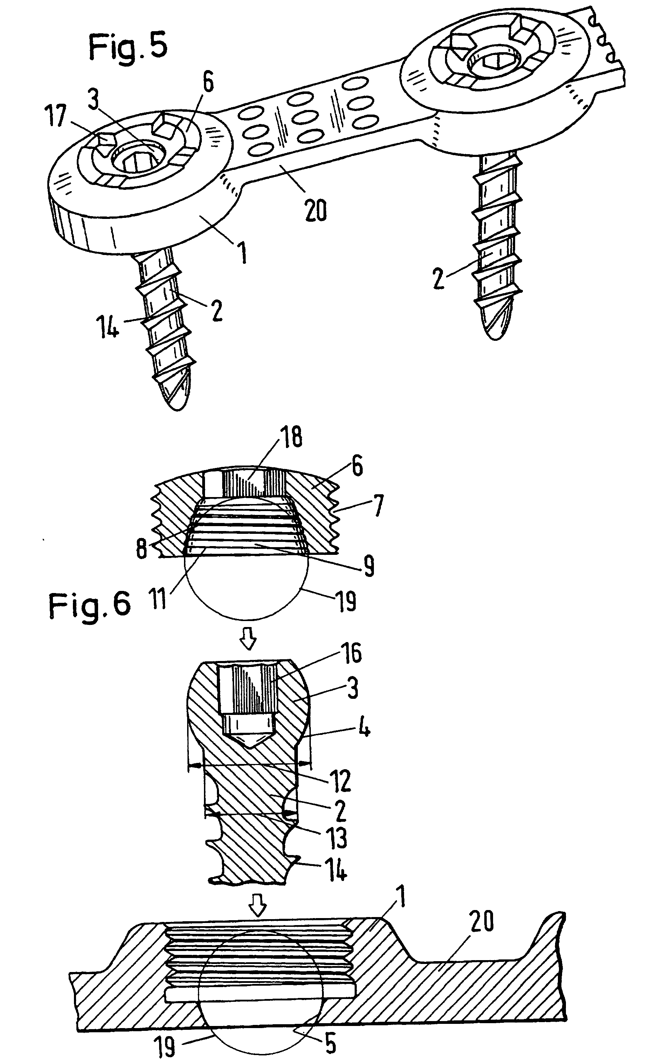

In Figur 5 ist ein verbindendes Zwischenstück 20 zwischen zwei Knochenschrauben 2 durch Verringerung des Querschnitts so geschwächt worden, dass es präoperativ bleibend verbogen werden kann.

In Figur 6 ist ein weiteres Ausführungsbeispiel gezeigt, wobei die gleichen Hinweiszeichen wie in den vorhergehenden Beispielen gelten. Osteosyntheseplatte 1, Knochenschraube 2 und Sicherungsschraube 6 sind in der Reihenfolge, in der sie montiert werden, übereinander gezeichnet. Zur besseren Verdeutlichung der Lage des Kugelkopfes 3 im montierten Zustand ist eine Kugel 19 mit gleichem Durchmesser an Platte 1 und Sicherungsschraube 6 angedeutet. Die Teile sind vergrössert, aber relativ zueinander im Massstab gezeichnet. Wenn man bespielsweise einen Durchmesser von 6 mm für diese Kugel 19 annimmt, wird erst bewusst, wie flach und sanft in den äusseren Uebergängen diese formschlüssige Verbindung gestaltet ist. Die Sicherungsschraube 6 besitzt einen Innensechskant 18 mit einer grösseren Schlüsselweite als der Innensechskant 16 des Kugelkopfes 3. Das Gewinde 14 der Knochenschraube 2 ist selbstschneidend wie in Figur 5.

Um die notwendigen Biegekräfte für die bleibende Verformung eines Zwischenstücks 20 intraoperativ aufzubringen, sind zwei Werkzeuge 22 (siehe Figuren 7, 8), welche einen stielartigen Handgriff und an ihrer Spitze ein angeformtes Kupplungsstück aufweisen. Das Kupplungsstück besitzt das gleiche Aussengewinde 7a wie eine Sicherungsschraube 6 und eine Anlagefläche 4 wie diejenige des Schraubenkopfes 3, um es zeitweilig mit der Platte 1 zu verbinden. Auf diese Weise können beidseitig von einem verformbaren Zwischenstück 20 Werkzeuge eingeschraubt und festgesetzt werden, die ein sicheres Einleiten der Biegekräfte ermöglichen.

In Figur 7 entspricht die Anpressfläche des Werkzeuges der Anlagefläche 4 eines kugeligen Schraubenkopfes 3.

In Figur 8 ist die Anpressfläche ringförmige auf der Oberseite der Platte 1 verteilt.

In Figur 9 ist eine Distanzschraube 23 an einer nicht von Knochenschrauben besetzten Schraubenaufnahme der Platte 1 eingeschraubt. Die Distanzschraube umfasst einen vorstehenden Stempel 24, der mit seinem Ueberstand über die Unterseite der Platte 1 deren Distanz zum Knochen festlegt. Die Einschraubtiefe der Distanzschraube 25 kann unterschiedlich gewählt werden oder durch einen Anschlag begrenzt werden.

## Patentansprüche

1. Osteosyntheseplatte mit mehreren Knochenschrauben (2), welche einen Kopf (3) mit kugelförmiger Anlagefläche (4) aufweisen, um beim Eindrehen in einem vorgegebenen Winkelbereich α unter einem Schrägwinkel auf einer ringförmigen, annähernd sphärischen Fläche (5) der Osteosyntheseplatte (1) anzuliegen, und mit einer Sicherungsschraube (6), welche in der Richtung auf die ringförmige, sphärische Fläche (5) mit einem Aussengewinde (7) in der Osteosyntheseplatte einschraubbar ist, um den Kopf der Knochenschraube (2) dort anzupressen, wobei
die kugelförmige Anlagefläche (4) mit gleichem Radius in den Bereich der Sicherungsschraube (6) fortgesetzt ist, **dadurch gekennzeichnet, dass** die Sicherungsschraube (6) eine konische Vertiefung (8) mit einem Innengewinde (9) aufweist, welches in der gleichen Richtung wie das Aussengewinde verläuft und die gleiche Steigung s besitzt, um mit dem Innengewinde (9) in seiner eigenen Spur eine Gewinderille (10) am Kopf (3) der Knochenschraube zu erzeugen, was wesentlich zur Erhöhung der Biegesteifigkeit zwischen Knochenschraube und Osteosyntheseplatte beiträgt.

2. Osteosyntheseplatte nach Anspruch 1, **dadurch gekennzeichnet, dass** das Innengewinde (9) mehrgängig ausgeführt ist, um die Anzahl der am Kopf (3) angreifenden Gewindespitzen (11) zu erhöhen.

3. Osteosyntheseplatte nach einem der Ansprüche 1 oder 2, **dadurch gekennzeichnet, dass** der Werkstoff der Sicherungsschraube (6) härter als der Werkstoff des Kopfes (3) der Knochenschraube (2) ist, um die Gewinderille (10) durch plastische Verformung im Schraubenkopf (3) zu erzeugen.

4. Osteosyntheseplatte nach einem der Ansprüche 1 oder 2, **dadurch gekennzeichnet, dass** die konische Vertiefung (8) quer zu den Gewinderillen (10) mit Nuten versehen ist, um Schneidkanten und Späneaufnahmeräume für das Schneiden der Gewinderille des Kugelkopfes zu bilden.

5. Osteosyntheseplatte nach einem der Ansprüche 1 bis 4, **dadurch gekennzeichnet, dass** der Durchmesser (12) des Kugelteils des .. Schraubenkopfes (3) um weniger als 40 % grösser als der Aussendurchmesser (13) des Gewindes (14) der Knochenschraube (2) ist.

6. Osteosyntheseplatte nach einem der Ansprüche 1 bis 5, **dadurch gekennzeichnet, dass** die konische Vertiefung (8) einen halben Konuswinkel β < 20° aufweist.

7. Osteosyntheseplatte nach Anspruch 6, **dadurch gekennzeichnet, dass** der halbe Konuswinkel β = 15° beträgt.

8. Osteosyntheseplatte nach einem der Ansprüche 1 bis 7, **dadurch gekennzeichnet, dass** die Gewindesteigung der Sicherungsschraube s ≤ 1 mm, vorzugsweise 0,75 mm beträgt.

9. Osteosyntheseplatte nach einem der Ansprüche 1 bis 8, **dadurch gekennzeichnet, dass** die Unterseite der Platte im Bereich der Knochenschraube (2) einen vorstehenden Kragen (15) zur Auflage auf einer Knochenoberfläche aufweist.

10. Osteosyntheseplatte nach einem der Ansprüche 1 bis 9, **dadurch gekennzeichnet, dass** die Knochenschrauben (2) mit Zwischenräumen (20) verteilt sind und dass die Zwischenräume (20) zwischen den Knochenschrauben (2) so geschwächt sind, dass sie dort mit Werkzeugen von Hand plastisch verbiegbar sind.

11. Osteosyntheseplatte nach Anspruch 10 mit einem Werkzeug (22), welches einen stielartigen Griff aufweist und in einer Spitze mit einem angeformten Kupplungsstück endet, welches ein Aussengewinde (7a) wie dasjenige der Sicherungsschraube (6) und eine Anlagefläche aufweist, um nach Einschrauben und Festsetzen des Werkzeugs (22) auf der Platte (1) das Zwischenstück (20) mittels des Handgriffes in eine gewünschte Richtung zu verbiegen.

12. Osteosyntheseplatte nach einem der Ansprüche 1 bis 11 mit einer Distanzschraube (23), welche über die Unterseite der Platte (1) vorsteht und nach dem Sichern von benachbarten Knochenschrauben (2) durch Sicherungsschrauben (6) mit einem Schraubendreher entfernbar ist.

## Claims

1. An osteosynthetic plate having a plurality of bone screws (2) which have a head (3) with a spherical contact face (4) in order to lie on a ring-shaped, approximately spherical surface (5) of the osteosynthetic plate (1) on being screwed in at an oblique angle within a predetermined angular range α and having a securing screw (6) with an external thread (7) which can be screwed into the osteosynthetic plate in the direction towards the ring-shaped spherical surface (5) in order to press onto the head of the bone screw (2) there, wherein the spherical contact face (4) is continued with the same radius into the region of the securing screw (6), **characterised in that** the securing screw (6) has a conical recess (8) with an internal thread (9) which extends in the same direction as the external thread and has the same pitch s in order to produce a thread groove (10) at the head (3) of the bone screw with the internal thread (9) in its own track, which substantially contributes to the increasing of the bending stiffness between the bone screw and the osteosynthetic plate.

2. An osteosynthetic plate in accordance with claim 1, **characterised in that** the internal thread (9) is designed with multiple turns in order to increase the number of the thread crests (11) engaging at the head (3).

3. An osteosynthetic plate in accordance with any one of claims 1 or 2, **characterised in that** the material of the securing screw (6) is harder than the material of the head (3) of the bone screw (2) in order to produce the thread groove (10) by plastic deformation in the screw head (3).

4. An osteosynthetic plate in accordance with any one of claims 1 or 2, **characterized in that** the conical recess (8) is provided with grooves transversely to the thread grooves (10) in order to form cutting edges and chip receiving spaces for the cutting of the thread groove of the spherical head.

5. An osteosynthetic plate in accordance with any one of claims 1 to 4, **characterised in that** the diameter (12) of the spherical part of the screw head (3) is larger than the external diameter (13) of the thread (14) of the bone screw (2) by less than 40%.

6. An osteosynthetic plate in accordance with any one of claims 1 to 5, **characterised in that** the conical recess (8) has a half cone angle β < 20°.

7. An osteosynthetic plate in accordance with claim 6, **characterised in that** the half cone angle β = 15°.

8. An osteosynthetic plate in accordance with any one of claims 1 to 7, **characterised in that** the thread pitch of the securing screw amounts to s ≤ 1 mm, preferably to 0.75 mm.

9. An osteosynthetic plate in accordance with any one of claims 1 to 8, **characterised in that** the lower side of the plate has a projecting collar (15) in the region of the bone screw (2) for support on a bone surface.

10. An osteosynthetic plate in accordance with any one of claims 1 to 9, **characterised in that** the bone screws (2) are distributed with intermediate spaces (20); and **in that** the intermediate spaces (20) are attenuated between the bone screws (2) such that they are plastically bendable by hand with tools there.

11. An osteosynthetic plate in accordance with claim 10 comprising a tool (22) which has a shaft-like handle and ends in a tip having a shaped coupling piece which has an external thread (7a) like that of the securing screw (6) and a contact face in order to bend the intermediate piece (20) in a desired direction by means of the handle after the screwing and fixing of the tool (22) to the plate (1).

12. An osteosynthetic plate in accordance with any one of claims 1 to 11 comprising a spacer screw (23) which projects beyond the lower side of the plate (1) and can be removed using a screwdriver after the securing of adjacent bone screws (2) by securing screws (6).

## Revendications

1. Plaque d'ostéosynthèse avec plusieurs vis à os (2) qui présentent une tête (3) avec une surface d'appui (4) de forme sphérique, afin de venir s'appuyer lors du vissage dans une plage angulaire α prédéfinie sous un angle incliné sur une surface (5) de forme annulaire, approximativement sphérique, de la plaque d'ostéosynthèse (1), et avec une vis de blocage (6), qui peut être vissée en direction de la surface (5) de forme annulaire, sphérique, avec un filetage extérieur (7) dans la plaque d'ostéosynthèse, afin de presser sur la tête de la vis à os (2) à cet endroit, sachant que la surface d'appui (4) de forme sphérique est poursuivie avec le même rayon dans la zone de la vis de blocage (6), **caractérisé en ce que** la vis de blocage (6) présente une cavité (8) conique avec un filetage intérieur (9), qui est agencé dans la même direction que le filetage extérieur et à la même pente s, afin de générer avec le filetage interne (9) dans sa propre trace une cannelure de filetage (10) sur la tête (3) de la vis à os, ce qui contribue de façon importante à l'amélioration de la rigidité de flexion entre la vis à os et la plaque d'ostéosynthèse.

2. Plaque d'ostéosynthèse selon la revendication 1, **caractérisé en ce que** le filetage interne (9) est réalisé avec plusieurs pas, afin d'augmenter le nombre des extrémités de filetage (11) s'appliquant sur la tête (3).

3. Plaque d'ostéosynthèse selon l'une quelconque des revendications 1 ou 2, **caractérisé en ce que** le matériau de la vis de blocage (6) est plus dur que le matériau de la tête (3) de la vis à os (2), afin de générer la cannelure de filetage (10) par déformation plastique dans la tête de vis (3).

4. Plaque d'ostéosynthèse selon l'une quelconque des revendications 1 ou 2, **caractérisée en ce que** la cavité (8) conique est pourvue de rainures transversalement aux cannelures de filetage (10) afin de former des arêtes de coupe et des espaces de logement de copeaux pour le coupage de la cannelure de filetage de la tête sphérique.

5. Plaque d'ostéosynthèse selon l'une quelconque des revendications 1 à 4, **caractérisée en ce que** le diamètre (12) de la partie sphérique de la tête de vis (3) est supérieur de moins de 40 % au diamètre extérieur (13) du filetage (14) de la vis à os (2).

6. Plaque d'ostéosynthèse selon l'une quelconque des revendications 1 à 5, **caractérisée en ce que** la cavité (8) conique présente un demi-angle de cône β < 20°.

7. Plaque d'ostéosynthèse selon la revendication 6, **caractérisée en ce que** le demi-angle de cône β = 15°.

8. Plaque d'ostéosynthèse selon l'une quelconque des revendications 1 à 7, **caractérisée en ce que** la pente de filetage de la vis de blocage est s ≤ 1 mm, de préférence égale à 0,75 mm.

9. Plaque d'ostéosynthèse selon l'une quelconque des revendications 1 à 8, **caractérisée en ce que** le côté inférieur de la plaque présente dans la zone de la vis à os (2) une collerette (15) saillante pour l'appui sur une surface d'os.

10. Plaque d'ostéosynthèse selon l'une quelconque des revendications 1 à 9, **caractérisée en ce que** les vis à os sont réparties avec des interstices (20) et **en ce que** les interstices (20) entre les vis à os (2) sont si affaiblis qu'ils peuvent être pliés au niveau plastique manuellement avec des outils à ces endroits.

11. Plaque d'ostéosynthèse selon la revendication 10 avec un outil (22) qui présente une poignée en forme de manche et se termine sur une extrémité avec une pièce d'accouplement formée dessus, qui présente un filetage externe (7a) comme celui de la vis de blocage (6) et une surface d'appui, afin de plier la pièce intermédiaire (20) au moyen de la poignée dans une direction souhaitée après le vissage et la fixation de l'outil (22) sur la plaque (1).

12. Plaque d'ostéosynthèse selon l'une quelconque des revendications 1 à 11 avec une vis d'espacement (23), qui dépasse du côté inférieur de la plaque (1) et peut être enlevée après le blocage de vis à os (2) voisines par des vis de blocage (6) avec un tournevis.
